# EUROPEAN PATENT APPLICATION

(11) **EP 1 840 035 A1**
(43) Date of publication of application: **03.10.2007**
(21) Application number: 07105235.1
(22) Date of filing: 29.03.2007
(51) Int. Cl.: B65B 55/06, A61L 2/06

(54) **Method for sterilising and depyrogenating bottles and similar containers, and apparatus for carrying out said method**

(30) Priority: 31.03.2006 IT BO20060223
(71) Applicant: Neri S.p.A., 50031 Barberino del Mugello FI (IT)
(72) Inventor: Calamandrei, Alessandro, 51017 Pescia (PT) (IT)
(74) Representative: Dall'Olio, Giancarlo

(57) **Abstract**

The present method for sterilising and depyrogenating containers comprises the following operating phases: stepwise moving at least a batch of containers (1), so as to enter them in an input station (11); removing liquid residuals from the containers (1) surface; stepwise moving said batch of containers (1) so as they leave the input station (11) and enter in a sterilising and depyrogenating station (21); thermally insulating the sterilising station (21) with respect to the outer environment; affecting the batch of containers (1) with a laminar flow of hot air, so as to raise the containers temperature up to a predetermined temperature value; keeping the batch of containers (1) inside the sterilising station (21) for a predetermined period of time, at least equal to that needed to obtain a certifiable sterilisation and depyrogenation of said batch of containers (1); stepwise moving the batch of containers (1) so as they leave the sterilising station (21) and they enter an output station (31); affecting the batch of containers (1) with a laminar air flow for lowering the containers (1) temperature to a desired value.

## Description

The present invention relates to a method for sterilizing and/or depyrogenating bottles and similar containers, by example for pharmaceutical use, and to a plant for carrying out said method.

It is known, in the automatic packaging machines industry, that some containers, as bottles, phials, syringes, particularly fit for receiving products that need a sterile environment for their preservation, as by example the pharmaceutical products, must be sterilized before filling them with the same products, in order to prevent any product contamination.

For this purposes there are known sterilization apparatus consisting of a tunnel provided with a belt conveyor for continuously transporting the bottles through subsequent processing stations of the sterilization apparatus.

In a first processing station (or input station) the bottles, which come from a washing station, are submitted to a laminar flow of air heated to approximately 70°C , in order to remove therefrom any residual washing liquid.

A second processing station, or sterilizing station, the bottles, is placed downstream of the input station, and it comprises a thermally insulated chamber inside which the temperature is kept at a value as high as around 350 °C, by suitable heating means. All the containers cross the aforesaid chamber so as any kind of micro-organisms are removed from inside the same containers, thus avoiding any possibility of containers contamination. Moreover, most of the potentially dangerous chemical agents are subject to chemical disgregation by the heat action, as organic toxins and other substances. During this operating phase, the containers are subject to the action of a hot laminar air flow.

The third station, so called cooling station, is placed downstream of the sterilizing station. It is fit to gradually lower the containers temperature, until they reach a predetermined temperature, before they are transported toward the subsequent filling stations.

As it is absolutely necessary that no contaminating agents coming from the outer environment reach the inner chambers of the above three operating stations, each of them is normally provided with HEPA absolute filters, having a suitable filtering grade and an efficiency of 99.999 DOP.

More particularly, it is known that the aforesaid chambers are pressurized to progressive pressures, by example according to a step profile. That means that each chamber is pressurized to a greater pressure with respect to the previous one, so as to prevent contaminant agents eventually residing in a chamber to flow into the downstream chamber. In any case, the input and output chambers are pressurized to a pressure higher than the atmospheric pressure.

It is often necessary that the whole filling and packaging process is carried out in a sterile environment; therefore, the situation of the three operating stations (and, more particularly, of the sterilizing station) as far as it regards the presence of contaminating or polluting agents, becomes essential both for obtaining an optimal sterilization grade across the whole drug production and packaging process, as well as for obtaining the certification for the above process and for the apparatus that carry out the same process.

The most important drawback of the production and packaging plants of known art is that it is not possible to know exactly the temperature attained by each container inside the sterilizing station. ln this latter, in fact, there are provided some fixed temperature probes, fit to measure the air temperature at some predefined positions inside the station. Sometimes, temporary temperature probes can be used for measuring the temperature attained by other locations inside the station chamber during approval or inspection tests. Nevertheless these are sample measurements, which don't guarantee that the same results are attained during the normal operations, when the containers continuously flow across the stations.

For the above stated reasons it is not possible to ensure that in the plants of known art that the air temperature, as measured with the fixed probes, is the same as the real temperature inside the containers.

A further drawback in the known packaging plants resides in that the belt conveyor on which the containers are transported crosses all the three operating stations, which therefore cannot be completely closed and insulated from the other operating stations nor from the outer environment. This structural characteristic feature, combined with the drawback described above, makes somewhat difficult to obtain the plant certification. In fact, the most important reason is that, since the sterilizing station is not completely closed, it is not possible to measure the exact temperature attained by all the containers processed inside the same sterilizing station, and also to ensure that the temperature inside the sterilizing station is homogeneously above a critical temperature.

It is an object of the present invention to provide a method for sterilising and/or depyrogenating containers which allows an apparatus that carries out said method to be able to easily obtain the certification for the containers sterilising and depyrogenating process.

A further object of the invention is to provide a method that allows all the containers to reach a homogeneous, predefined sterilising temperature during the sterilising and depyrogenating process.

A further object of the invention is to provide a method for sterilising and depyrogenating containers which allows an apparatus that carries out the aforesaid method to easily obtain a plant certification.

Again an object of the present invention is to provide a method consisting of simple and quick operating phases.

A further object of the present invention is to provide an apparatus for carrying out the aforesaid sterilising and depyrogenating method that allows to obtain the certification for the sterilisation and depyrogenation for the processed containers, and thus to obtain the complete certification for the whole drug packaging process.

Again another object of the present invention is to provide an apparatus for sterilising and depyrogenating containers that allows all the processed containers to reach, during their sterilising and depyrogenating process, a homogeneous predefined temperature.

A further object of the present invention is to provide an apparatus that ensure a high reliability standard in all the operating conditions.

All the above stated objects are attained according to the contents of the appended Claims.

The characteristic features of the present invention will be explained in the following, wherein a preferred, but not exclusive, embodiment, together with a structure variant thereof, with reference to the enclosed drawings, wherein:
- Figure 1 shows schematically a partially sectional side view of an apparatus for sterilising and depyrogenating containers fit to carry out a method according to the present invention, in a first operating phase;
- Figure 2 shows a schematic, partially sectional side view of the apparatus of figure 1 in a subsequent operating phase;
- Figure 3 shows schematically a structural variant of the apparatus of figure 1 and figure 2.

Referring now to Figures 1 and 2 it is indicated with numeral 100 an apparatus for sterilising and/or depyrogenating containers, by example containers for pharmaceutical products, made according to the present invention.

The aforesaid apparatus 100 comprises conveying means (10,20,30) operated stepwise, fit to convey a number of containers 1. On an active branch of said conveying means lays a first batch of containers 1. The above operating means preferably consists of a first closed belt conveyor 10, a second closed belt conveyor 20 and a third belt conveyor 30, which are synchronously operated, and which are placed so as to have their active branch laying on the same plane.

The sterilising apparatus 100 comprises an input station 11. The first belt conveyor 10 crosses this latter in its lower part. The input station 10 is fit to dry the containers 1 coming, by example, from an upstream washing station. The input station 11 is provided with air flow generating means, of a known type, fit to generate a laminar air flow directed toward the first containers batch 1, in order to remove all the residual liquid substances from their surface, by evaporating same residual liquid.

A sterilising and /or depyrogenating station 21, mounted downstream of the above input station 11, is crossed in its lower part by the second belt conveyor 20. The sterilising and/or depyrogenating station 21 is provided with known air generating means, fit to generate a laminar hot air flow directed toward the containers 1 for elevating their temperature up to a predetermined value T*. The sterilising and/or depyrogenating station 21 comprises a thermally insulated chamber 23, which is crossed by the second belt conveyor 20. The aforesaid chamber 23 is provided with two side openings 24, respectively a left side opening 24A and a right side opening 24B, counterfacing one each other and fit to respectively allow the input and the output of the containers 1 from the same chamber 23.

A closure means 22 is mounted on the outer side walls of the thermally insulated chamber 23, in order to insulate this latter completely with respect to the outer environment. More particularly, closure means 22 comprises a pair of doors 24, respectively a left door 24A and a right door 24B, which close their corresponding side openings 24, left side opening 24A and right side opening 24b, substantially for the period of time comprised between two subsequent stepwise shifts of the second belt conveyor 20.

Suitable temperature detectors 29, which are provided inside the sterilising and depyrogenating chamber 23, are able to measure, in several different points in the chamber 23, the temperature of hot air affecting the containers 1.

More particularly, a plurality of temperature detectors 29 can be provided in the chamber 23, each of those is positioned so as to counterface a defined container belonging to a batch being sterilised inside the thermally insulated chamber 23.

An output station 31, mounted downstream to the sterilising and depyrogenating station 21, is crossed in its lower part by the third belt conveyor 30. the output station 31 is provided with air flow generating means of a known type, fit to generate a laminar air flow directed toward the containers 1, in order to progressively lower their temperature.

Each of the above mentioned operating stations (11,21,31) comprises corresponding absolute filters HEPA, mounted according to known techniques, having an adequate air filtering grade, and an efficiency of preferably 99,999 DOP, in order to prevent any polluting substance from being ejected to the open air.

More particularly, the three operating stations (11,21,31) are progressively pressurized, by example according to a step pressure profile. That is, each operating station is pressurized, with respect to the previous one, so as to prevent polluting substances eventually present therein from flowing to the previous operating station. The input station 11 and the output station 31 are naturally pressurized with respect to the outer environment.

Some operating modes of the present apparatus 100 will be now described in the following, even if they are very intuitive and easy to understand.

For this purpose, reference will be made to a start-up configuration wherein the doors (22A, 22B) are raised, so as to allow the input and output of the containers 1 to/from the chamber 23.

A first batch of containers 1 is placed on the first belt conveyor 10 which, is moved stepwise, synchronously with respect to the second belt conveyor 20 and to the third belt conveyor 30, until all the containers of the batch are inside the input station 11, where they can be dried.

A subsequent, synchronous stepwise moving of all the belt conveyors, first conveyor 10, second conveyor 20 and third conveyor 30, allows the first batch of containers 1 to move from the input station 11 to the sterilising and depyrogenating station 21.

While the first batch of containers is leaving the input station 11 and reaches the sterilising and depyrogenating station 21, a second batch of containers 1 is moved from the washing station to the input station 11.

The doors 22A, 22B are then driven to a closed position, so as to close the openings 24A, 24B, and the chamber 23 of the sterilising station 21 is thermally insulated from the outside. Subsequently, the temperature inside the thermally insulated chamber 23 is raised up to a predefined value T*. Said predefined value T* is a temperature value that guarantees an optimum sterilisation and depyrogenation of containers 1.

While the containers 1 stay inside the chamber 23, since this latter is thermally insulated with respect to the outer environment, heat can rise homogeneously inside the chamber 23. In this way, the temperature measured by temperature detectors 29 corresponds in fact to the chamber temperature attained statically, and thus to the temperature attained by all the containers 1 that are present inside the same chamber 23.

After a predetermined period of time Δt* during which the containers 1 stay at a temperature T*, at least equal to the period of time that is necessary to optimally sterilising and depyrogenating the containers 1, and therefore to obtain the corresponding certification, the doors 22A,22B are opened, thus restoring the air communication between the chamber 23 and the other operating stations, input station 11 and output station 31.

The subsequent stepwise moving of the first belt conveyor 10, second belt conveyor 20 and third belt conveyor 30 allows the first batch of containers 1 to pass from the second belt conveyor 20 to the third belt conveyor 30, and from the sterilising and depyrogenating chamber 23 to the output station 31.

The above stepwise moving of the belt conveyors 10,20,30 also allows the aforesaid second batch of containers 1 from the input station 11 to the sterilising station 21 and to the thermally insulated chamber 23, as well as a third batch of containers 1 to be moved on the first belt conveyor 10 to the input station 11.

A subsequent stepwise moving of the belt conveyors 10,20,30 allows the first batch of containers to be moved out from the output station 31 and to be conveyed toward a subsequent downstream operating stations, by example toward a filling station, for filling the containers 1 with corresponding products.

Figure 3 shows a structural variant of the sterilising apparatus 100, wherein the aforesaid conveying means consists of a single, stepwise operated, belt conveyor 50.

The characteristic operating phases of the method according to the present invention, carried out by means of the above described sterilising apparatus 100 will be resumed in the following, even if they should be already clear after the above description.

The operating phases of the sterilising and depyrogenating method of a batch of containers 1, subsequently to a washing phase, substantially comprise:
- stepwise moving said batch of containers 1, until all the containers are inside the input station 11;
- keeping the batch of containers 1 inside the input station 11 for a predetermined period of time Δt, sufficient to allow the residual liquid remaining in the containers 1 to be removed by a laminar air flow that affects the same batch of containers 1;
- stepwise moving the batch of containers 1, until they are all moved out from the input station 11 and they are all moved inside the sterilising and depyrogenating station 21;
- thermally insulating the chamber 23 of the sterilising and depyrogenating station 21 with respect to the outer environment;
- affecting the batch of containers 1 by a laminar flow of hot air, so as to allow the temperature inside the chamber 23, and therefore the containers temperature, to rise up to a predefined value T*;
- keeping the batch of containers 1 inside the chamber 23 at the predefined temperature value T* for a predetermined period of time Δt*, said period of time Δt being at least equal to the time necessary to attain an optimal containers sterilisation and depyrogenation, and therefore to obtain the corresponding certification;
- stepwise moving the aforesaid batch of containers 1 until they are moved out from the sterilising and depyrogenating station 21 and they are moved in the output station 31 ;
- affecting the batch of containers 1 with an air laminar flow, fit to allow the temperature of the same containers 1 to lower.

A verification that the temperature T* has been reached and kept for the right time can be carried out by means of the temperature detectors 29 placed inside the sterilising and depyrogenating chamber 23 for measuring the current air temperature inside the chamber 23 in several different points.

From the above description is now clear that the movable doors 22, provided in the sterilising and depyrogenating station 21 for closing and thermally insulating the same sterilising station 21 with respect to the outer environment, permit to obtain a great advantage in the sterilising operation, as they allow said operation to be carried out statically.

This structural detail allows a predetermined temperature T* to be certainly reached inside the thermally insulated chamber 23 of the sterilising and depyrogenating station 21, as well as the heat inside the same chamber 23 to be distributed in an homogeneous and uniform way. This makes very easy to obtain the official certifications for the described apparatus.

This occurs thanks to the possibility of measure certainly the temperature attained by all the containers 1 present inside the chamber 23.

Advantageously, the temperature detectors 29 are placed inside the sterilising chamber 23 so as to face corresponding containers 1 of the batch staying inside the same chamber 23, and allow a precise and reliable temperature measuring for all the same containers 1. By means of the sterilising apparatus of the present invention is therefore possible to be certain that the containers 1 stay inside the hot chamber 23 for a minimum period of time, and also that all the containers 1 stay at a predetermined temperature for a minimum period of time. This allows the apparatus certification could be obtained easily and with no doubt, as it is demonstrable that all the containers 1 have been subject to the same heating process. Therefore, there is a double certainty when carrying out the above sterilising method with the described apparatus 100. First of all, a time certainty, that means that all the containers 1 will stay inside the sterilising chamber 23 for a predetermined period of time. Moreover, during this predetermined period of time, all the containers will be subject to a temperature at least equal to a predetermined value.

The sterilising apparatus 100 according to the present invention allows therefore all the containers 1 to be sterilised and depyrogenated in an optimum and demonstrable, and then also easily certifiable, way.

Advantageously, the three belt conveyors 10,20,30 are moved stepwise and they extend for the longitudinal extension of their respective operating stations 11,21,31: this characteristic feature allows the doors to be lowered completely, and the corresponding openings to be completely closed. The sterilising chamber 23 can then be completely thermally insulated with respect to the outer environment.

Moreover, the three operating stations 11,21,31 can advantageously work simultaneously on several batches of containers 1, so as to improve the apparatus throughput.

It has to be highlighted that the operating phases of the present method are extremely fast and simple.

## Claims

1. Method for sterilising and/or depyrogenating containers previously subject to a washing phase, **characterised in that** it comprises the following operating phases:
- stepwise moving at least one batch of containers (1), until all the containers are inside an input station (11);
- keeping said batch of containers (1) inside said input station (11) for a predetermined period of time (Δt), sufficient to allow the residual liquid remaining in said containers (1) to be removed by a laminar air flow that affects the same batch of containers (1);
- stepwise moving said batch of containers (1), until they are all moved out from said input station (11) and they are all moved inside a sterilising chamber (23) of a sterilising and depyrogenating station (21);
- thermally insulating said chamber (23) of the sterilising and depyrogenating station (21) with respect to the outer environment;
- affecting said batch of containers (1) by a laminar flow of hot air, so as to allow the temperature inside said chamber (23), and therefore the containers temperature, to rise up to a predefined value (T*);
- keeping said batch of containers (1) inside said chamber (23) at said predefined temperature value (T*) for a predetermined period of time (Δt*), said period of time (Δt*) being at least equal to the time necessary to attain an optimal containers sterilisation and depyrogenation, and therefore to obtain the corresponding certification;
- stepwise moving said batch of containers (1) until they are moved out from said sterilising and depyrogenating station (21) and they are moved to said output station (31);
- affecting said batch of containers (1) with a laminar air flow, fit to allow the temperature of the same containers (1) to lower.

2. Method according to Claim 1, wherein all said operating stations (11,21,31) are full of corresponding batches of containers (1), **characterised in that** the period of time of permanence of said batches of containers (1) inside their corresponding operating stations (11,21,31) corresponds to a predefined value, depending on the processing cycle necessities.

3. Apparatus for sterilising and/or depyrogenating containers, mounted downstream to a washing station fit to wash said containers (1), comprising conveying means (10,20,30,50) fit to move said containers (1) across said operating stations (11,21,31) and affecting, subsequently, an input station (11) provided with air flow generating means for generating a laminar air flow toward said containers (1) for removing from their surface all the residual washing liquid; a sterilising and/or depyrogenating station (21), fit to sterilise and/or depyrogenate said containers (1), provided with air flow generating means for generating a laminar flow of hot air toward said containers (1) for raising the containers (1) temperature; an output station (31), fit to cool said containers (1), provided with air flow generating means for generating a laminar air flow toward said containers (1) for lower their temperature, said apparatus (100) being **characterised in that** said conveying means (10,20,30,50) are moved stepwise, and **in that** the period of time that elapses between two subsequent conveyor moving operations is such that the batch of containers (1) staying in said input station (11) becomes dried, the batch of containers (1) staying in said sterilising and/or depyrogenating station (21) becomes sterilised and/or depyrogenated, and the batch of containers (1) staying in said output station (31) becomes cooled.

4. Apparatus according to Claim 3, **characterised in that** said conveying means comprises a first belt conveyor (10), a second belt conveyor (20) and a third belt conveyor (30), operated synchronously.

5. Apparatus according to Claim 4, **characterised in that** said first belt conveyor (10), second belt conveyor (20) and third belt conveyor (30) respectively affect the lower part of said input station (11), said sterilising and/or depyrogenating station (21) and said output station (31).

6. Apparatus according to Claim 3, **characterised in that** said conveying means comprises a single belt conveyor (50) operated stepwise.

7. Apparatus according to one of the previous Claims 3 to 6, **characterised in that** said sterilising and/or depyrogenating station (21) comprises at least a thermally insulated chamber (23), provided with a pair of counter-facing side openings, a left side opening (24A) and a right side opening (24B), respectively fit to allow said batches of containers (1) to enter said chamber (23) and to leave said chamber (23), and **in that** there is provided closure means (22), associated to said sterilising and/or depyrogenating station (21), fit to close said openings (24A, 24B) and therefore to thermally insulate said chamber (23) with respect to the outer environment.

8. Apparatus according to Claim 7, **characterised in that** said closure means (22) comprises a pair of doors, respectively a left door (22A) and a right door (22B), fit to respectively engage said left side opening (24A) and right side opening (24B) during the period of time comprised between two subsequent stepwise movings of said conveying means (10,20,30), so as to thermally insulate said chamber (23) with respect to the outer environment.
